# EUROPÄISCHE PATENTSCHRIFT

(11) **EP 0 466 092 B1**
(45) Veröffentlichungstag und Bekanntmachung des Hinweises auf die Patenterteilung: **24.08.1994**
(21) Anmeldenummer: 91111400.7
(22) Anmeldetag: 09.07.1991
(51) Int. Cl.: A61K 9/70, A61K 9/02, A61L 15/16

(54) **Pharmazeutisches, vaginal zu applizierendes Präparat**
Vaginal pharmaceutical preparation
Préparation pharmaceutique pour application vaginale

(30) Priorität: 10.07.1990 CH 2294/90
(43) Veröffentlichungstag der Anmeldung: 15.01.1992
(73) Patentinhaber: Laboratoire Lucchini S.A., 1204 Genève (CH)
(72) Erfinder: Heusser, Jean, CH-8134 Adliswil (CH); Martin, Michel, CH-1206 Genève (CH)
(74) Vertreter: Zink-Wild, Markus Peter

(56) Entgegenhaltungen:
- GB-A- 1 196 678
- CHEMICAL ABSTRACTS, Band 98, Nr. 26, Juni 1983, Seite 381, Zusammenfassung Nr. 221811r, Columbus, Ohio, US; & JP-A-58 032 816 (SHIN-ETSU CHEMICAL INDUSTRY) 25-02-1983
- PATENT ABSTRACTS OF JAPAN, Band 7, Nr. 114 (C-166)[1259], 18. Mai 1983, Seite 12 C 166; & JP-A-58 032 816 (SHINETSU KAGAKU KOGYO K.K.) 25-02-1983

## Beschreibung

Die vorliegende Erfindung betrifft ein pharmazeutisches, vaginal zu applizierendes Präparat.

Für die pharmazeutische Behandlung von vaginalen Krankheiten werden vor allem Vaginaltabletten, Salben, Gele, Schäume und Ovuli verwendet. Jede dieser Darreichungsformen hat Vor- und Nachteile. Von den Nachteilen seien erwähnt: schlechter Zerfall des Präparates, Fremdkörpergefühl, schlechte Verteilung des jeweiligen Wirkstoffes auf der Vaginalschleimhaut, komplizierte Dosierung, beispielsweise mittels eines Applikators. Ein Schaum kann ein unangenehmes Gefühl verursachen und kann deshalb aus objektiven und/oder subjektiven Gründen abgelehnt werden. Ferner ist ein sogenannter C-Film für die Schwangerschaftsverhütung bekannt.

Siehe dazu:
- A.S. Lichtmann, V. Davajan, D. Tucker, C-Film: A new vaginal contraceptive. Contraception Vol. 8.4 pp 291-297 1973.
- The Johns Hopkins University, Baltimore MD, USA, New developments in vaginal contraception. Population Reports Vol XII.1 serie H Nr 7 1984.
- Organisation Mondiale de la Santé, Contraception mécanique et spermicides. Leur rôle en planification familiale. Organisation Mondiale de la Santé 1987.

Vorrichtungen, respektive Filme zur Empfängnisverhütung sind beschrieben in:
- GB-A-1 196 678
- Chemical Abstracts, 98, Nr. 26, Juni 1983, Seite 381, Nr. 221 811 r
- Patent Abstracts of Japan, Band 7, Nr. 114 (C-166) [1259] vom 18. Mai 1983.

Es ist ein Ziel der vorliegenden Erfindung, eine neue galenische Form, nämlich ein Film, für die dosierte Abgabe der Wirkstoffe zur lokalen Behandlung von sexuell übertragbaren, resp. übertragenen Krankheiten , und/oder Vaginalaffektionen zur Verfügung zu stellen. Diese neue galenische Form soll die Wirkstoffe auf der Vaginalschleimhaut relativ rasch, gut und gleichmässig verteilen. Ebenso soll die Stabilität der im Film vorhandenen aktiven Verbindungen erhöht werden.

Diese Ziele werden mit dem erfindungsgemässen Präparat in hervorragender Weise erreicht.

Das erfindungsgemässe pharmazeutische, vaginal zu applizierende Präparat ist dadurch gekennzeichnet, dass es wenigstens einen in Wasser löslichen Polyvinylalkohol, wenigstens einen Bestandteil A, ausgewählt aus der Gruppe, bestehend aus Netzmitteln, nichtionischen oberflächenaktiven Mitteln und Dispergiermitteln, sowie wenigstens einen Wirkstoff B zur lokalen Behandlung von sexuell übertragbaren, resp. übertragenen Krankheiten, und/oder Vaginalaffektionen, und gegebenenfalls einen oder mehrere Hilfsstoff(e), homogen verteilt enthält, und in der Form eines Filmes mit einer Schichtdicke von 0,05 bis 0,5 mm, insbesondere 0,06 bis 0,2 mm, vorzugsweise 0,07 bis 0,15 mm, vorliegt.

Bevorzugte Ausführungsformen dieser Erfindung sind in den abhängigen Ansprüchen definiert.

Im Folgenden wird die mögliche Herstellung des erfindungsgemässen Präparates beschrieben.

Ein in Wasser löslicher Polyvinylalkohol , beispielsweise Poval 205 der Firma Kuraray Co.Ltd, Japan, wird mit wenigstens einem genannten Bestandteil A, beispielsweise ein Gemisch aus wenigstens einem Nonoxynol, wie etwa Nonoxynole der Formel 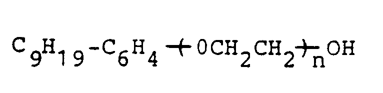
worin n eine ganze Zahl, vorzugsweise die Zahl 9 oder 10, bedeutet,und Glycerin, und mit Wasser vermischt. Danach wird diese Mischung erwärmt, beispielsweise auf eine Temperatur von etwa 90°C. Es ist bevorzugt, die abgekühlte Lösung, beispielsweise bei einer Temperatur von etwa 50°C, zu filtrieren. Falls der Wirkstoff B und die gegebenenfalls zu verwendenden Hilfsstoffe in Wasser löslich sind, werden sie in Wasser gelöst, und vorzugsweise vor der Filtration der Mischung zugegeben. Falls der Wirkstoff B und die gegebenenfalls zu verwendenden Hilfsstoffe in Wasser nicht löslich sind, werden sie nach der genannten Filtration der Mischung, vorzugsweise unter Rühren, zugegeben, beispielsweise in der Form einer Suspension oder Emulsion, wobei wässrige Suspensionen oder Emulsionen bevorzugt sind. Wasser- in-Oel-Mikroemulsionen und Oel-in-Wasser-Mikroemulsionen sind ebenfalls anwendbar. Es ist auch möglich, einen in Wasser nicht löslichen Wirkstoff B und/oder Hilfsstoff in fester Form, vorzugsweise unter Rühren, der filtrierten Mischung zuzugeben, insbesondere dann, wenn die Suspension homogenisiert wird. Es ist auch möglich, den Wirkstoff B und/oder Hilfsstoff in wenigstens einem organischen Lösungsmittel gelöst der Mischung zuzugeben. Bei dieser Art der Zugabe darf es zwischen organischem Lösungsmittel und Wasser keine Phasentrennung geben. Wenn nötig kann zum Korrosionsschutz von Filmherstellungsvorrichtungen die Filmgiesslösung gepuffert werden.

Die erwähnte Filtration wird durchgeführt, um gegebenenfalls vorhandene Verunreinigungen, wie etwa wasserunlösliche Polyvinylalkohol - Polymerisate, Staub und andere Fremdpartikel, zu entfernen. Bei absolut sauberen und einheitlichen Produkten könnte auf eine Filtration verzichtet werden. Die Filmgiesslösung muss homogen sein. Gegebenenfalls vorhandene Luftblasen können mittels Stehenlassen der Lösung oder mittels Vakuum oder mittels leichtem Umrühren entfernt werden.

Diese Filmgiesslösung kann nun auf mehrere Arten in herkömmlicher Art zu einem Film verarbeitet werden.

Bei einer ersten Art wird die Filmgiesslösung ansatzweise in eine Wanne , welche die jeweils gewünschten Dimensionen hat, gegossen. Danach werden das Wasser und gegebenenfalls vorhandene organische Lösungsmittel mittels Trocknung entfernt. Das Trocknen kann beispielsweise mittels Heissluft, aus einem geeigneten Föhn stammend, oder einer geeigneten Heizlampe, beispielsweise eine Infrarotlampe, erfolgen. Die jeweils angewendete Temperatur muss derart sein, dass kein Bestandteil durch Wärmeeinwirkung chemisch umgewandelt,insbesondere zersetzt,wird.Der Film kann nun noch geprägt werden, wodurch sich dessen Oberfläche und dessen Griffigkeit erhöhen. Danach wird der Film geschnitten und konfektioniert, z.B. in Sachets abgepackt,und kann bei Bedarf durch eine geeignete Bestrahlungsmethode steril gemacht werden.

Bei einer zweiten Art wird die Filmgiesslösung kontinuierlich in einer Filmgiessvorrichtung verarbeitet. Dabei wird die Filmgiesslösung durch eine Schlitzdüse auf ein laufendes Band gegossen. Dieser Filmträger ist normalerweise ein auf Hochglanz poliertes Chromstahlband. Beim Giessen muss die Viskosität der Filmgiesslösung derart sein, dass sie noch fliesst aber nicht stehen bleibt.
Die jeweils verwendete Viskosität ist u.a. vom Maschinentyp abhängig. Das mit der Filmgiesslösung beschichtete laufende Band wird durch einen Trocknungstunnel geführt, worin das Wasser und das gegebenenfalls vorhandene organische Lösungsmittel entfernt werden. Die im Trocknungstunnel angewendete Temperatur muss derart sein, dass kein Bestandteil durch Wärmeeinwirkung chemisch umgewandelt, insbesondere zersetzt, wird. Am Ende des Trocknungstunnels enthält der Film nur noch wenige Gew.-% an Wasser, beispielsweise etwa 5 Gew.-% Wasser. Die jeweils gewünschte Filmdicke kann durch einen Computer überwacht und gesteuert werden. Ist beispielsweise der Vorlauf eines Filmes zu dünn, so wird automatisch die Schlitzdüse etwas geöffnet. Ist der Film hingegen zu dick, so wird die Schlitzdüse etwas geschlossen. Betreffend Prägung, Schnitt und Konfektion wird auf die obigen diesbezüglichen Ausführungen verwiesen.

Der Wirkstoff B zur lokalen Behandlung von sexuell übertragbaren, resp. übertragenen Krankheiten und/oder Vaginalaffektionen ist insbesondere ein Wirkstoff zur lokalen Behandlung von bakteriellen oder viralen Infektionen oder ein Wirkstoff zur lokalen Behandlung von durch Pilze oder Trichomonaden verursachte Erkrankungen. Als Beispiele seien genannt: Benzalkoniumchlorid, Neomycine, wie etwa Neomycin-B-sulfat, Polymyxine, wie etwa Polymyxin-B-sulfat, Econazol, Econazolnitrat und Metronidazol. Diese und weitere aktive Verbindungen, einschliesslich ein Placentaextrakt, können mit einem Nonoxynol vermischt sein, beispielsweise mit Nonoxynol der Formel: 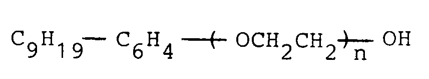
worin n eine ganze Zahl, vorzugsweise die Zahl 9 oder 10, bedeutet. Nonoxynol-9 und Nonoxynol-10 sind bevorzugt, insbesondere dann, wenn diese Verbindungen die Vorschriften gemäss USP 22 erfüllen.

Das erfindungsgemässe Präparat ist sehr einfach zu applizieren. Es kann, zugeschnitten auf die gewünschte Grösse, beispielsweise 5x5 cm, beispielsweise mit dem Finger in die Vagina eingeführt werden. Ein Fremdkörpergefühl wird, wenn überhaupt, nur in den ersten Minuten nach dem Einführen empfunden. Durch die Einwirkung von Körperwärme und/oder von den in der Vagina vorhandenen Sekrete kommt (kommen) der (die) im Film enthaltene(n) Wirkstoff(e) im Vergleich zu andern, vor allem festen galenischen Formen besser und gleichmässiger zur Wirkung. Der zu erzielende pharmazeutische Effekt hängt von der jeweils verwendeten Wirkstoffkombination ab.

Die nachfolgenden Beispiele dienen der Illustration der vorliegenden Erfindung.

### Beispiel 1

### Vaginalpräparat gegen bakterielle Infektionen

Ein Gemisch aus 4,53 kg Polyvinylalkohol Poval 205 der Firma Kuraray Co, Ltd., Japan, 360 g Glycerin, 2,16 kg Nonoxynol-9 und 600 g Benzalkoniumchlorid wurde in 17,35 kg Wasser unter Rühren langsam auf eine Temperatur von 90°C erwärmt. Nachdem sich alle Komponenten gelöst hatten wurde die leicht trübe Lösung abgekühlt und bei einer Temperatur von 50°C filtriert. Das klare Filtrat wurde bei einer Temperatur von 45°C während 30 Minuten stehen gelassen, wonach im Gemisch keine Luftblasen mehr vorhanden waren. Mit dieser Filmgiesslösung wurde auf einer Filmgiessmaschine ein homogener Film mit einer Dicke von 0,09 Millimeter hergestellt. Die Filmgiesslösung wurde bei einer Temperatur von 40°C bis 50°C gegossen.

### Beispiel 2

### Vaginalpräparat gegen Trichomonaden

Ein Gemisch aus 453 mg Polyvinylalkohol Poval 205 der Firma Kuraray Co, Ltd.,Japan, 36 mg Glycerin, 216 mg Nonoxynol-9 und 100 mg Metronidazol wurde in 10 ml Wasser gelöst und unter Rühren auf eine Temperatur von 90°C erwärmt. Die leicht trübe Lösung wurde abgekühlt und bei einer Temperatur von 50°C filtriert. Dieses Filtrat wurde in eine rechteckige Wanne (5x10 cm) gegossen und mit einer Wärmelampe (Biccatherm-Lampe) getrocknet. Man erhielt einen homogenen Film mit einer Dicke von 0,075 Millimeter.

### Beispiel 3

### Vaginalpräparat für die Bekämpfung von Pilzen

Ein Gemisch aus 3,01 kg Polyvinylalkohol Poval 205 der Firma Kuraray Co, Ltd., Japan, 240 g Glycerin und 1,0 kg Nonoxynol wurde in 13,35 kg Wasser unter Rühren langsam auf eine Temperatur von 90°C erwärmt. Die leicht trübe Lösung wurde abgekühlt und bei einer Temperatur von 50°C filtriert. Zu diesem klaren Filtrat wurde unter Rühren eine Aufschlämmung von 400 g fein zermahlenem Econazol in 2,0 kg Wasser bei einer Temperatur von 35°C hinzugegeben. Dieses Gemisch wurde bei einer Temperatur von 35°C während 30 Minuten leicht gerührt, wonach im Gemisch keine Luftblasen mehr vorhanden waren. Mit dieser Filmgiesslösung wurde auf einer Filmgiessmaschine ein homogener Film mit einer Dicke von 0,07 Millimeter hergestellt. Die Filmgiesslösung wurde bei einer Temperatur von 35°C gegossen.

## Patentansprüche (Patentansprüche für folgende(n) Vertragsstaat(en): AT, BE, CH, DE, DK, FR, GB, GR, IT, LI, NL, SE)

1. Pharmazeutisches, vaginal zu applizierendes Präparat, dadurch gekennzeichnet, dass es wenigstens einen in Wasser löslichen Polyvinylalkohol, wenigstens einen Bestandteil A, ausgewählt aus der Gruppe, bestehend aus Netzmitteln, nichtionischen oberflächenaktiven Mitteln und Dispergiermitteln, sowie wenigstens einen Wirkstoff B zur lokalen Behandlung von sexuell übertragbaren, resp. übertragenen Krankheiten, und/oder Vaginalaffektionen, und gegebenenfalls einen oder mehrere Hilfsstoff(e),homogen verteilt enthält, und in der Form eines Filmes mit einer Schichtdicke von 0,05 bis 0,5 mm, insbesondere 0,06 bis 0,2 mm, vorzugsweise 0,07 bis 0,15 mm, vorliegt.

2. Präparat nach Anspruch 1, dadurch gekennzeichnet, dass der Wirkstoff B ein Wirkstoff zur lokalen Behandlung von bakteriellen oder viralen Infektionen, beispielsweise Benzalkoniumchlorid, ein Neomycin, wie etwa Neomycin-B-sulfat, oder ein Polymyxin, wie etwa Polymyxin-B-sulfat, oder ein Wirkstoff zur lokalen Behandlung von durch Pilze oder Trichomonaden verursachte Erkrankungen, beispielsweise Econazol, Econazolnitrat, Metronidazol, oder ein Wirkstoff zur Behandlung von Vaginalschleimhautaffektionen ist, beispielsweise ein Placentaextrakt, wobei diese Wirkstoffe gegebenenfalls mit wenigstens einem Nonoxynol vermischt sein können, wie etwa Nonoxynole der Formel 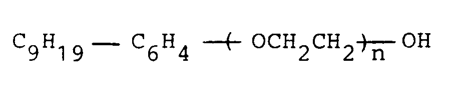 worin n eine ganze Zahl, vorzugsweise die Zahl 9 oder 10, bedeutet.

3. Präparat nach einem der Ansprüche 1 bis 2, dadurch gekennzeichnet, dass der Bestandteil A ausgewählt ist aus einwertigen Alkoholen, beispielsweise einem Nonoxynol, wie etwa Nonoxynol-9, Nonoxynol-10, und mehrwertigen Alkoholen, beispielsweise Propylenglycol, Glycerin, und insbesondere ein Gemisch aus einem Nonoxynol und Glycerin ist.

4. Präparat nach einem der Ansprüche 1 bis 3, dadurch gekennzeichnet, dass es 50 bis 70 Gew.-%, insbesondere 55 bis 65 Gew.-%, Polyvinylalkohol, 15 bis 35 Gew.-%, insbesondere 20 bis 30 Gew.-%, Nonoxynol, 3 bis 8 Gew.-%, insbesondere 5 Gew.-%, Glycerin, und bis zu 15 Gew.-% Wirkstoff B enthält, wobei die Summe aller Bestandteile, einschliesslich der gegebenenfalls vorhandenen Hilfsstoffe, jeweils 100 Gew.-% ergibt.

5. Präparat nach einem der Ansprüche 1 bis 4, dadurch gekennzeichnet, dass die Hilfsstoffe ausgewält sind aus Stabilisatoren, Weichmachern, Puffern, Antioxidantien, Parfum und Farbstoffen, und dass sie jeweils in wirksamen Konzentrationen enthalten sind.

6. Präparat nach einem der Ansprüche 1 bis 5, dadurch gekennzeichnet, dass die Oberfläche des Filmes durch Prägung vergrössert ist.

## Patentansprüche (Patentansprüche für folgende(n) Vertragsstaat(en): ES)

1. Verfahren zur Herstellung eines pharmazeutischen, vaginal zu applizierenden Präparates, dadurch gekennzeichnet, dass man wenigstens einen in Wasser löslichen Polyvinylalkohol, wenigstens einen Bestandteil A, ausgewählt aus der Gruppe, bestehend aus Netzmitteln, nichtionischen oberflächenaktiven Mitteln und Dispergiermitteln, sowie wenigstens einen Wirkstoff B zur lokalen Behandlung von sexuell übertragbaren, resp. übertragenen Krankheiten, und/oder Vaginalinfektionen, und gegebenenfalls einen oder mehrere Hilfsstoff(e), mit Wasser, und gegebenenfalls wenigstens einem organischen Lösungsmittel, vermischt und unter Erwärmung, beispielsweise auf eine Temperatur von etwa 90°C, homogen verteilt, die erhaltene Mischung giesst und die vorhandenen Lösungsmittel entfernt.

2. Verfahren nach Anspruch 1, dadurch gekennzeichnet, dass der Wirkstoff B ein Wirkstoff zur lokalen Behandlung von bakteriellen oder viralen Infektionen, beispielsweise Benzalkoniumchlorid, ein Neomycin, wie etwa Neomycin-B-sulfat, oder ein Polymyxin, wie etwa Polymyxin-B-sulfat, oder ein Wirkstoff zur lokalen Behandlung von durch Pilze oder Trichomonaden verursachte Erkrankungen, beispielsweise Econazol, Econazolnitrat, Metronidazol, oder ein Wirkstoff zur Behandlung von Vaginalschleimhautaffektionen ist, beispielsweise ein Placentaextrakt, wobei diese Wirkstoffe gegebenenfalls mit wenigstens einem Nonoxynol vermischt werden, wie etwa Nonoxynole der Formel 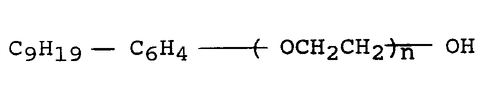 worin n eine ganze Zahl, vorzugsweise die Zahl 9 oder 10 bedeutet.

3. Verfahren nach einem der Ansprüche 1 bis 2, dadurch gekennzeichnet, dass der Bestandteil A ausgewählt ist aus einwertigen Alkoholen, beispielsweise einem Nonoxynol, wie etwa Nonoxynol-9, Nonoxynol-10, und mehrwertigen Alkoholen, beispielsweise Propylenglycol, Glycerin, und insbesondere ein Gemisch aus einem Nonoxynol und Glycerin ist.

4. Verfahren nach einem der Ansprüche 1 bis 3, dadurch gekennzeichnet, dass das Mischverhältnis so eingestellt wird, dass das Präparat 50 bis 70 Gew.-%, insbesondere 55 bis 65 Gew.-%, Polyvinylalkohol, 15 bis 35 Gew.-%, insbesondere 20 bis 30 Gew.-%, Nonoxynol, 3 bis 8 Gew.-%, insbesondere 5 Gew.-%, Glycerin, und bis zu 15 Gew.-% Wirkstoff B enthält, wobei die Summe aller Bestandteile, einschliesslich der gegebenenfalls vorhandenen Hilfsstoffe, jeweils 100 Gew.-% ergibt.

5. Verfahren nach einem der Ansprüche 1 bis 4, dadurch gekennzeichnet, dass man die Hilfsstoffe aus Stabilisatoren, Weichmachern, Puffern, Antioxidantien, Parfum und Farbstoffen auswählt, und dass die Hilfsstoffe jeweils in wirksamen Konzentrationen enthalten sind.

6. Verfahren nach einem der Ansprüche 1 bis 5, dadurch gekennzeichnet, dass die Mischung vor dem Giessen filtriert wird.

7. Verfahren nach einem der Ansprüche 1 bis 6, dadurch gekennzeichnet, dass das Präparat in der Form eines Filmes mit einer Schichtdicke von 0,05 bis 0,5 mm, insbesondere 0,06 bis 0,2 mm, vorzugsweise 0,07 bis 0,15 mm, vorliegt.

8. Verfahren nach einem der Ansprüche 1 bis 7, dadurch gekennzeichnet, dass die Oberfläche des Filmes durch Prägung vergrössert wird.

## Claims (Claims for the following Contracting State(s): AT, BE, CH, DE, DK, FR, GB, GR, IT, LI, NL, SE)

1. A pharmaceutical, vaginal applicable preparation, characterized in that it contains, homogeneously sub-divided, at least one in water soluble polyvinyl alcohol, at least one component A, selected from the group consisting of wetting agents, non-ionic surface active agents and dispersing agents, as well as at least one active component B for the local treatment of sexual transmissible or transmitted, respectively, diseases, and/or for vaginal affections, and occasionally one or more auxiliary agent(s), and in that it is in the form of a film having a thickness of the layer of from 0.05 to 0.5 mm, especially from 0.06 to 0.2 mm, preferably from 0.07 to 0.15 mm.

2. The preparation according to claim 1, characterized in that the active component B is an active component for the local treatment of bacterial or viral infections, for example benzalkoniumchloride, a neomycin, such as neomycin-B-sulfate, or a polymyxin, such as polymyxin-B-sulfate, or an active component for the local treatment of diseases caused by fungus' or trichomonas, for example econazol, econazolnitrate, metronidazol, or an active component for the treatment of vaginal mucosa affections, for example a placenta extract, whereby these active components occasionally may be mixed with at least one nonoxynol, such as nonoxynols of the formula 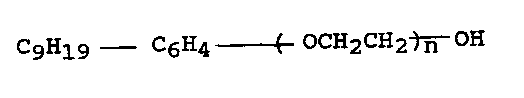 wherein n is an integer, preferably 9 or 10.

3. The preparation according to one of claims 1 to 2, characterized in that the component A is selected from monovalent alcohols, for example a nonoxynol, such as nonoxynol-9, nonoxynol-10, and polyvalent alcohols, for example propylene glycol, glycerin, and is especially a mixture of a nonoxynol and glycerin.

4. The preparation according to one of claims 1 to 3, characterized in that it contains from 50 to 70 % by weight, especially from 55 to 65 % by weight, of a polyvinyl alcohol, from 15 to 35 % by weight, especially from 20 to 30 % by weight, of nonoxynol, from 3 to 8 % by weight, especially 5 % by weight, of glycerin, and up to 15 % by weight of active component B, whereby the total of all components, including the occasionally present auxiliary agent(s), gives always 100 % by weight.

5. The preparation according to one of claims 1 to 4, characterized in that the auxiliary agents are selected from stabilizers, plasticizers, buffers, antioxidants, perfumes and dyes, and in that they are present always in active concentrations.

6. The preparation according to one of claims 1 to 5, characterized in that the surface of the film is increased by means of embossing.

## Claims (Claims for the following Contracting State(s): ES)

1. A process for preparing a pharmaceutical, vaginal applicable preparation, characterized in that at least one in water soluble polyvinyl alcohol, at least one component A, selected from the group consisting of wetting agents, non-ionic surface active agents and dispersing agents, as well as at least one active component B for the local treatment of sexual transmissible or transmitted, respectively, diseases, and/or for vaginal affections, and occasionally one or more auxiliary agent(s), are mixed with water, and occasionally with at least one organic solvent, and are homogeneously sub-divided under heating, for example to a temperature of about 90°C, and in that the so obtained mixture is poured and in that the present solvents are removed.

2. The process according to claim 1, characterized in that the active component B is an active component for the local treatment of bacterial or viral infections, for example benzalkoniumchloride, a neomycin, such as neomycin-B-sulfate, or a polymyxin, such as polymyxin-B-sulfate, or an active component for the local treatment of diseases caused by fungus' or trichomonas, for example econazol, econazolnitrate, metronidazol, or an active component for the treatment of vaginal mucosa affections, for example a placenta extract, whereby these active components occasionally may be mixed with at least one nonoxynol, such as nonoxynols of the formula 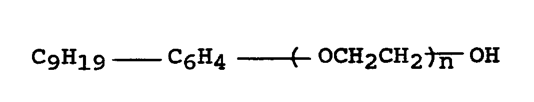 wherein n is an integer, preferably 9 or 10.

3. The process according to one of claims 1 to 2, characterized in that the component A is selected from monovalent alcohols, for example a nonoxynol, such as nonoxynol-9, nonoxynol-10, and polyvalent alcohols, for example propylene glycol, glycerin, and is especially a mixture of a nonoxynol and glycerin.

4. The process according to one of claims 1 to 3, characterized in that the mixing proportion is ajusted in such a way, that the preparation contains from 50 to 70 % by weight, especially from 55 to 65 % by weight, of a polyvinyl alcohol, from 15 to 35 % by weight, especially from 20 to 30 % by weight, of nonoxynol, from 3 to 8 % by weight, especially 5 % by weight, of glycerin, and up to 15 % by weight of active component B, whereby the total of all components, including the occasionally present auxiliary agent(s), gives always 100 % by weight.

5. The process according to one of claims 1 to 4, characterized in that the auxiliary agents are selected from stabilizers, plasticizers, buffers, antioxidants, perfumes and dyes, and in that they are present always in active concentrations.

6. The process according to one of claims 1 to 5 characterized in that the mixture is filtrated before it is poured.

7. The process according to one of claims 1 to 6, characterized in that the preparation is in the form of a film having a thickness of the layer of from 0.05 to 0.5 mm, especially from 0.06 to 0.2 mm, preferably from 0.07 to 0.15 mm.

8. The process according to one of claims 1 to 7, characterized in that the surface of the film is increased by means of embossing.

## Revendications (Revendications pour l'(les) Etat(s) contractant(s) suivant(s): AT, BE, CH, DE, DK, FR, GB, GR, IT, LI, NL, SE)

1. Préparation pharmaceutique à application vaginale, caractérisée en ce qu'elle contient un mélange homogène d'au moins un alcool polyvinylique hydrosoluble, d'au moins une composante A, choisie dans le groupe se composant d'agents mouillants, d'agents tensio-actifs non-ioniques et d'agents dispersants, ainsi que d'au moins une substance active B pour le traitement local de maladies sexuellement transmissibles ou transmises, et/ou d'affections vaginales et, éventuellement, d'un ou plusieurs adjuvants et se présente sous la forme d'un film d'une épaisseur de couche de 0,05 à 0,5 mm, en particulier 0,06 à 0,2 mm, de préférence 0,07 à 0,15 mm.

2. Préparation selon la revendication 1, caractérisée en ce que la substance active B est une substance active pour le traitement local d'infections bactériennes ou virales, par exemple du chlorure de benzalconium, une néomycine, comme par exemple la néomycine-B-sulfate ou une polymyxine, comme par exemple la polymyxine-B-sulfate ou une substance active pour le traitement local d'affections engendrées par des champignons ou des trichomonas, par exemple de l'éconazole, du nitrate d'éconazole, du métronidazole ou une substance active pour le traitement d'affections des muqueuses vaginales, par exemple un extrait de placenta, ces substances actives pouvant éventuellement être mélangées avec au moins un nonoxynol, comme par exemple les nonoxynols de formule
C₉H₁₉ ― C₆H₄ ― (OCH₂CH₂)ₙ ― OH
où n est un nombre entier, de préférence 9 ou 10.

3. Préparation selon l'une quelconque des revendications 1 à 2, caractérisée en ce que la composante A est choisie parmi des monoalcools, par exemple un nonoxynol, comme par exemple le nonoxynol-9, le nonoxynol-10 et des polyalcools, par exemple du propylèneglycol, la glycérine et est en particulier un mélange d'un nonoxynol et de glycérine.

4. Préparation selon l'une quelconque des revendications 1 à 3, caractérisée en ce qu'elle contient 50 à 70 % en poids, en particulier 55 à 65 % en poids d'alcool polyvinylique, 15 à 35 % en poids, en particulier 20 à 30 % en poids de nonoxynol, 3 à 8 % en poids, en particulier 5 % en poids de glycérine, et jusqu'à 15 % en poids de substance active B, la somme de toutes les composantes, y compris les adjuvants éventuellement présents, donnant respectivement 100 % en poids.

5. Préparation selon l'une quelconque des revendications 1 à 4, caractérisée en ce que les adjuvants sont choisis parmi les stabilisateurs, les plastifiants, les tampons, les antioxydants, les parfums et les colorants et en ce qu'ils sont respectivement contenus dans des concentrations efficaces.

6. Préparation selon l'une quelconque des revendications 1 à 5, caractérisée en ce que la surface du film est étendue par estampage.

## Revendications (Revendications pour l'(les) Etat(s) contractant(s) suivant(s): ES)

1. Procédé de fabrication d'une préparation pharmaceutique à application vaginale, caractérisé en ce qu'au moins un alcool polyvinylique hydrosoluble, au moins une composante A, choisie dans le groupe se composant d'agents mouillants, d'agents tensio-actifs non-ioniques, d'agents dispersants, ainsi qu'au moins une substance active B pour le traitement local de maladies sexuellement transmissibles ou transmises, et/ou d'affections vaginales et, éventuellement, un ou plusieurs adjuvants sont mélangés à de l'eau et, éventuellement, à au moins un solvant organique et répartis de manière homogène en chauffant par exemple à une température d'environ 90° C, le mélange obtenu est coulé et les dissolvants présents sont éliminés.

2. Procédé selon la revendication 1, caractérisé en ce que la substance active B est une substance active pour le traitement local d'infections bactériennes ou virales, par exemple du chlorure de benzalconium, une néomycine, comme par exemple la néomycine-B-sulfate ou une polymyxine, comme par exemple la polymyxine-B-sulfate ou une substance active pour le traitement local d'affections engendrées par des champignons ou des trichomonas, par exemple de l'éconazole, le nitrate d'éconazole, du métronidazole, ou une substance active pour le traitement d'affections des muqueuses vaginales, par exemple un extrait de placenta, ces substances actives pouvant éventuellement être mélangées à au moins un nonoxynol, comme par exemple les nonoxynols de formule
C₉H₁₉ ― C₆H₄ ― (OCH₂CH₂)ₙ ― OH
où n est un nombre entier, de préférence 9 ou 10.

3. Procédé selon l'une quelconque des revendications 1 à 2, caractérisé en ce que la composante A est choisie parmi des monoalcools, par exemple un nonoxynol, comme par exemple le nonoxynol-9, le nonoxynol-10 et des polyalcools, par exemple le propylèneglycol, la glycérine et est en particulier un mélange d'un nonoxynol et de glycérine.

4. Procédé selon l'une quelconque des revendications 1 à 3, caractérisé en ce que le rapport de mélange est réglé de sorte que la préparation contienne 50 à 70 % en poids, en particulier 55 à 65 % en poids d'alcool polyvinylique, 15 à 35 % en poids, en particulier 20 à 30 % en poids, de nonoxynol, 3 à 8 % en poids, en particulier 5 % en poids de glycérine, et jusqu'à 15 % en poids de substance active B, la somme de toutes les composantes, y compris les adjuvants éventuellement présents, donnant respectivement 100 % en poids.

5. Procédé selon l'une quelconque des revendications 1 à 4, caractérisé en ce que les adjuvants sont choisis parmi les stabilisateurs, les plastifiants, les tampons, les antioxydants, les parfums et les colorants et en ce que les adjuvants sont respectivement contenus dans des concentrations efficaces.

6. Procédé selon l'une quelconque des revendications 1 à 5, caractérisé en ce que le mélange est filtré avant le coulage.

7. Procédé selon l'une quelconque des revendications 1 à 6, caractérisé en ce que la préparation se présente sous la forme d'un film d'une épaisseur de couche 0,05 à 0,5 mm, en particulier 0,06 à 0,2 mm, de préférence 0,07 à 0,15 mm.

8. Procédé selon l'une quelconque des revendications 1 à 7, caractérisé en ce que la surface du film est étendue par estampage.
